# EUROPEAN PATENT SPECIFICATION

(11) **EP 0 626 854 B1**
(45) Date of publication and mention of the grant of the patent: **15.07.1998**
(21) Application number: 91909862.4
(22) Date of filing: 02.05.1991
(51) Int. Cl.: A61K 31/74, A61K 31/725, A61K 38/48, A01N 37/18

(54) **ANTI-THROMBOGENIC AND/OR ANTI-MICROBIAL COMPOSITION**
ANTITHROMBOGENE UND/ODER ANTIMIKROBIELLE ZUSAMMENSETZUNG
COMPOSITION ANTITHROMBOGENE ET/OU ANTIMICROBIENNE

(30) Priority: 12.07.1990 US 551924; 28.02.1991 US 662452
(43) Date of publication of application: 07.12.1994
(73) Proprietor: STS Biopolymers, Inc., Rush, New York 14523 (US)
(72) Inventor: WHITBOURNE, Richard, James, Fairport, NY 14450 (US); MANGAN, Margaret, Anne, Rochester, NY 14620 (US)
(74) Representative: W.P. THOMPSON & CO.
(86) International application number: US9102868
(87) International publication number: WO9200747

(56) References cited:
- EP-A- 0 328 421
- WO-A-89/05138
- WO-A-90/03768
- US-A- 4 127 647
- US-A- 4 871 357
- Science, Vol. 142, "Heparin Bonding on Colloidal Graphite Surfaces", GOTT et al. (1963), pages 1297-1298, see the Abstract.
- Investigative Radiology, Vol. 6, July-August (1971), "A Simple Non-thrombogenic Coating", K. AMPLATZ, pages 280-289.
- Vol. XV Trans. Amer. Soc. Artif. Int. Organs (1969), GRODE et al.: "Nonthrombogenic Materials via a Simple Coating Process", pages 1-6.

## Description

This invention relates to pharmaceutical coating compositions, coated medical devices and methods of coating medical devices.

Many kinds of polymer compositions have been used in the field of medical supplies. These compositions have not always exhibited anti-thrombogenic, anti-microbial, or other biocompatible characteristics when used in prosthetic and therapeutic apparatuses for handling or being in contact with blood or blood components or other bioresponse under conditions where clotting would tend to occur, such as artificial blood vessels, catheters, artificial hearts, fluid drainage, suction/aspiration and artificial kidneys.

When blood is brought in contact with metal, glass, plastic or other similar surfaces, it tends to clot in a short time unless certain precautions are taken. One common precaution currently in widespread use is the treatment of the surface with heparin or with heparin reacted with quaternary ammonium compounds. Such heparin compounds are known to have anti-coagulant effects when in contact with blood. The presence of the aforementioned heparin compounds on the surface imparts anti-thrombogenic characteristics. However, previously known heparinization or compositions have not been adequate because of the short time of anti-thrombogenic activity, at most a few days in vivo (I. S. Hersch, et al, J. Biomed., Mater. Res. Symposium I, 99-104 (1971); K. Amplatz, "A Simple Non-Thrombogenic Coating", Invest. Radiology, July, August, 1971, Vol. 6) or because the anti-thrombogenic characteristic was reduced to a very low level in order to make it resistant to removal by reacting it with quaternary ammonium polymers (U.S. Patent 3,844,989).

An object of the present invention is to provide novel pharmaceutically-coated surfaces which contain pharmaceutical agents which are entrained in the surface in such a way as to be gradually released in vivo to provide effective action over a longer time than was previously possible when using these agents. Typical agents useful in this embodiment of the invention include penicillins, cephalosporins, aminoglycosides, quinolones, sulfonoamides, tetracyclines, etc. While effective anti-microbial agent concentrations are achieved near the coated device surface, low systemic levels result, unlike where systemic antibiotic administration is required to combat infections caused by an implanted device. The compositions of the invention provide excellent properties for use as medical materials for coatings on artificial blood vessels, catheters, artificial hearts, artificial kidneys, etc.

### SUMMARY OF THE INVENTION

EP-A-0328421 relates to infection-resistant compositions, medical devices and surfaces and methods of preparing and using the same, the polymers used being polyurethane, silicone and biodegradable polymers.

WO-A-90/03768 relates to biodegradable polymers which are copolymers of thermoplastic and thermosetting polymers.

WO-A-89/05138 relates to a method of forming bioerodible implants for controlled drug release, the used being selected from polyanhydrides, polyesters, polyorthoesters, polyamides, polyurethanes, polyacrylontriles and polyphosphazenes.

US-A-4127647 discloses a solution for use in spray drying, comprising 9-acetyl-3''-acetylmidecamycin dissolved in chloroform and containing hydroxypropylmethyl cellulose phthalate as a stabilizing substance.

### SUMMARY OF THE INVENTION

The present invention is defined in the claims of this specification, to which reference should now be made.

The pharmaceutical agent compositions (mixtures) of this invention comprise pharmaceutical agents that are not reacted with ionic surfactants, providing that they have the appropriate solubility profile namely that they are soluble in organic solvents. They may also contain some hydrophilic polymers, but the mixture would still be water-insoluble after coating and drying. The water-insoluble cellulose ester polymers of this invention range from hydrophobic polymers to ones that are fairly hydrophilic, but are nevertheless essentially water-insoluble after being coated on a substrate and dried. A single polymer or mixture(s) of different polymers may be used to accomplish the invention. The pharmaceutical agent may be mixed in a solution with the water-insoluble polymer, or it may be coated on top of a coating of the water-insoluble cellulose ester polymer(s), which is applied to the surface beforehand. In the latter case, a solvent must be added that is a mutual solvent for both the pharmaceutical agent and the water-insoluble cellulose ester polymer(s) so that some mixing occurs between the two layers. In still another case, it is possible to coat the pharmaceutical agent directly on the water-insoluble plastic surface, and incorporate a mutual solvent for both the plastic surface and the pharmaceutical agent, so that some mixing occurs between the plastic surface and the pharmaceutical agent.

Various combinations of these three systems would be obvious to one skilled in the art. The mixtures of the water-insoluble cellulose ester polymer(s) and pharmaceutical agents of this invention are substantially more resistant to removal or deactivation in human and animal body fluids such as blood or plasma than the pharmaceutical agents by themselves.

Typical examples of water-insoluble cellulose ester polymers suitable for use with the present invention are as follows: cellulose acetate, cellulose acetate butyrate, cellulose acetate propionate, and cellulose nitrate. Optionally, polyurethane resins including polyether and polyester grades may also be present. Exemplary of the polyurethane is the reaction product of 2,4-tolylene diisocyanate and position isomers thereof, 4,4'-diphenylmethane diisocyanate and position isomers thereof, polymethylenepolyphenyl isocyanate, or 1,5-naphthylene diisocyanate with 1,2-polypropylene glycol, polytetramethylene ether glycol, 1,4-butanediol, 1,4-butylene glycol, 1,3-butylene glycol, poly(1,4-oxybutylene)glycol, caprolactone, adipic acid esters, phthalic anhydride, ethylene glycol, 1,3-butylene glycol, 1,4-butylene glycol or diethylene glycol. Acrylic polymers such as ethyl and methyl acrylate and methacrylate; condensation polymers such as those produced by sulfonoamides such as toluenesulfonamide and aldehydes such as formaldehyde; and isocyanate compounds may optionally also be present. Exemplary of the isocyanate compounds are polymethylenepolyphenyl isocyanate, 4,4'-diphenylmethane diisocyanate and position isomers thereof, 2,4-tolylene diisocyanate and position isomers thereof, 3,4-dichlorophenyl diisocyanate and isoferrone isocyanate. Adducts or prepolymers of isocyanates and polyols such as the adduct of trimethylolpropane and diphenylmethane diisocyanate or tolylene diisocyanate are suitable. For further examples of polyisocyanates, see "Encyclopedia of Polymer Science and Technology", H. F. Mark, N. G. Gaylord and N. M. Bikales (eds.) (1969).

### DETAILED DESCRIPTION OF THE INVENTION

The compositions of the present invention are first dissolved in solvent mixtures that are co-solvents for the mixtures of non-volatile components and which allow compatible homogenous films of the components to be cast. Such films when dried will typically appear as a clear film or films of very slight turbidity indicating that the non-volatile components have been deposited in a substantially homogenous manner. Typical solvents comprise alcohols, ketones, esters, aromatics, pyrrollidones, carboxylic acids, amides, and other organic solvents used alone or in appropriate mixtures as required, and which bring about the basic compatibility of the non-volatile components to be expressed. Typical surfaces which can be coated include plastic, metal and glass.

In most cases, all the components of the coating composition are incorporated into a single solution so that the surface treatment can be accomplished with a single application. However, the treatment can also be applied in two steps. For example, the water-insoluble cellulose ester polymer(s) can be applied in one application and the pharmaceutical agent can be applied to the water-insoluble polymer. Some mutual solvent(s) for the water-insoluble polymer and the pharmaceutical agent that makes two components compatible should be included in the overcoat application to accomplish the objective of the invention. For example, dimethylacetamide (DMA) effectively accomplishes this objective as shown in Example 1. A variant on this approach would involve application of the water-insoluble cellulose ester polymer(s) followed by application of a solution containing some water-insoluble cellulose ester polymer and some pharmaceutical agent, some of which may also be added to the first application. Typical concentrations of pharmaceutical agent in the coating solutions range from about 0.1% to 20% by weight. Preferred concentrations range from 0.5% up to 4%. Use of higher concentrations of pharmaceutical agents in the solutions does not enhance performance and is therefore not very useful or desired. Lower concentrations than those disclosed above reduce the activity levels of the layers.

Typical concentrations of the water-insoluble cellulose ester polymers in the coating solution range from about 0.01% to 20% by weight. Preferred concentrations range from about 0.2% to 3%. Higher concentrations tend to mask the efficacy of the layers. Lower concentrations tend to allow the layer to be extracted more easily. The composition of the final coating may have the pharmaceutical agent present in a concentration of about 0.5 to 99.5 percent by weight with the balance of the composition comprising essentially the water-insoluble cellulose ester polymer.

### ANTI-THROMBOGENICITY TEST

The following in vitro test was used to evaluate anti-thrombogenicity: 10mm x 75mm glass test tubes were charged with 0.5 gm of reconstituted human plasma which had been kept refrigerated since collection. The test tubes were equilibrated in a 37°C incubator for 10-30 minutes. Next, 0.1 g of 0.10 M CaCl₂ was added, and the test tube was manually swirled to achieve complete mixing. Immediately after swirling, 4-1/2" (114 mm) long sections of 7 French tubing (either coated with one of the anti-thrombogenic systems of the present invention, or uncoated controls) were dropped into the plasma in each tube, taking care to ensure that the sample pieces were completely immersed in the plasma. The tubes were maintained in the 37°C incubator and were checked for clotting at one minute intervals by removing them from the incubator and tilting them. Before clotting, the liquid flows in the test tube, but it gels and does not flow once it has clotted. Typical clotting times for plasma containing untreated polyurethane tubing range from six minutes to 15 minutes. Samples made according to this invention prevent clotting in this test. It was found that if the plasma did not clot after standing overnight, it would usually not clot for up to four weeks. Therefore, tests were usually discontinued if they had not clotted after standing overnight. Typical samples prepared by this invention did not clot when tested before plasma extraction, and retained their anti-clotting activity after 28 or more days of extraction in plasma. Devices coated with heparin-benzalkonium chloride or heparin-tridodecylmethylammonium chloride do not clot when tested before extraction in plasma, but lose their anti-thrombogenicity after plasma extraction of two hours or less. Heparinized quaternary polymers (HQP), such as those prepared according to U.S. Patent 3,844,989 and used on catheters marked under the trademark ANTHRON by Toray Medical Co. Ltd., show only slight anti-thrombogenicity. For example, when tested against heparin-benzalkonium chloride (HBAC), the HBAC sample prevented clotting of the plasma overnight, while the control clotted in five minutes and the HQP sample clotted in seven minutes before plasma extraction, and showed no improvement in anti-thrombogenicity compared to the untreated polyurethane control after 12 hours of plasma extraction.

In the following examples, Examples 19 to 22 are in accordance with the present invention. The remaining examples are for comparison or reference purposes.

### Example 1

Polyurethane 7 French tubing was coated with a solution containing the following ingredients and dried for 20 minutes at 65°C.

| | |
|---|---|
| Polyvinylpyrrolidone | .006 g |
| Isopropanol | 1.0 g |
| Nitrocellulose | 1.6 g |
| Ethylacetate | 1.2 g |
| Rosin ester | .5 g |
| Butylacetate | 4.8 g |
| Dimethylacetamide | 1.5 g |
| Ethyl-3-ethoxy propionate | 6.1 g |

The tubing was then overcoated with a solution containing the following ingredients and then dried for 20 minutes at 65°C.

| | |
|---|---|
| Isopropanol | 9.85 g |
| Dimethylacetamide | 1.00 g |
| Heparin-benzalkonium chloride | .15 g |

This sample was compared to a sample of polyurethane tubing which was coated with heparin-benzalkonium chloride (1.8% w/v in isopropanol) as follows. The samples were dipped in a Gentian Violet dye solution and then rinsed in hot running water. The sample coated with heparin-benzalkonium chloride (HBAC) in isopropanol lost most of the surface dye stain in less than 20 seconds, indicating that most of the HBAC had been washed off. The sample of the present invention that had the nitrocellulose undercoat and contained DMA in the HBAC overcoat, retained the dye stain much longer indicating that it is much more resistant to removal.

### Example 2

Polyurethane 7 French tubing was coated with a solution consisting of:

| | |
|---|---|
| Methylethylketone | 5.0 g |
| Heparin-benzalkonium chloride | 0.33 g |
| Isopropanol | 3.7 g |
| Ethyl-3-ethoxy propionate | .6 g |
| Butyl acetate | .5 g |
| 1/2 sec. nitrocellulose | .16 g |
| Ethyl acetate | .1 g |
| Rosin ester | .05 g |

The samples were dried at 75°C for 30 minutes. Samples were then extracted in human plasma at 37°C for 7, 10, 21, or 28 days and then tested for anti-clotting properties. The following results were obtained.

| Sample | Clotting time |
|---|---|
| Uncoated control | 12 minutes |
| Above sample, without extraction in plasma | Did Not Clot |
| Above sample, after 7 days extraction in plasma | Did Not Clot |
| Above sample, after 10 days extraction in plasma | Did Not Clot |
| Above sample, after 21 days extraction in plasma | 24 minutes |
| Above sample, after 28 days extraction in plasma | 20 minutes |

The above results show that the samples are still exhibiting effective anti-clotting activity on the device surface where it is most needed and that clots are unlikely to form on the treated surfaces, even after 28 days of extraction. This level of anti-clotting activity is stronger even after 28 days of plasma extraction than the anti-clotting levels achieved under these test conditions with surfaces treated according to the compositions taught by U.S. Patent 3,844,989.

### Example 3

The following solution was coated on polyurethane 7 French tubing and dried at 75°C for 20 minutes.

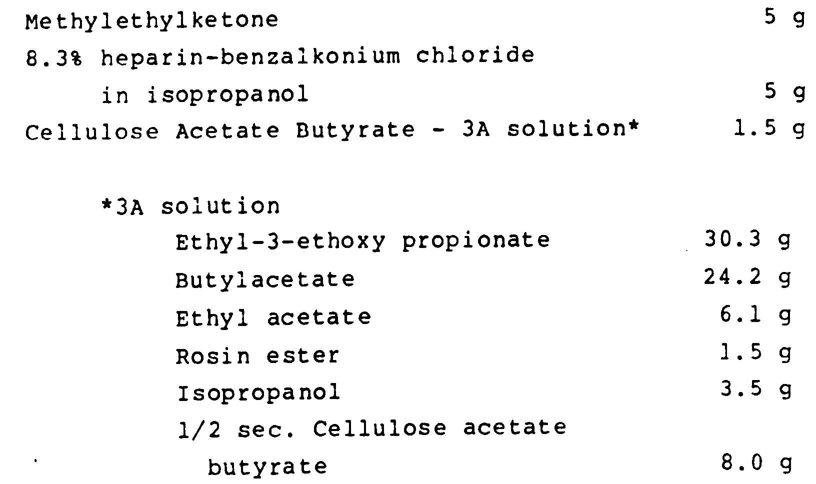

Coated samples were tested for anti-clotting activity, and also for resistance to removal by dyeing with Gentian Violet dye and then rinsing with hot running water. The sample was compared to a coating of heparin-benzalkonium chloride without any cellulose ester polymer additive.

Results: The sample did not clot in the clotting test. In the hot water rinse test, the heparin-benzalkonium chloride coating without cellulose resin was completely removed in a few seconds. Hot water rinsing did not remove the above coating which contained cellulose acetate butyrate polymer.

### Example 4

Polyurethane 7 French tubing was coated as in Example 3 except that cellulose acetate butyrate was replaced with cellulose acetate propionate. The sample was tested for anti-clotting activity and resistance to removal in hot water. Results were comparable to those with Example 3.

### Example 5

Polyurethane 7 French tubing was coated with the following solution and dried at 80°C for 20 minutes.

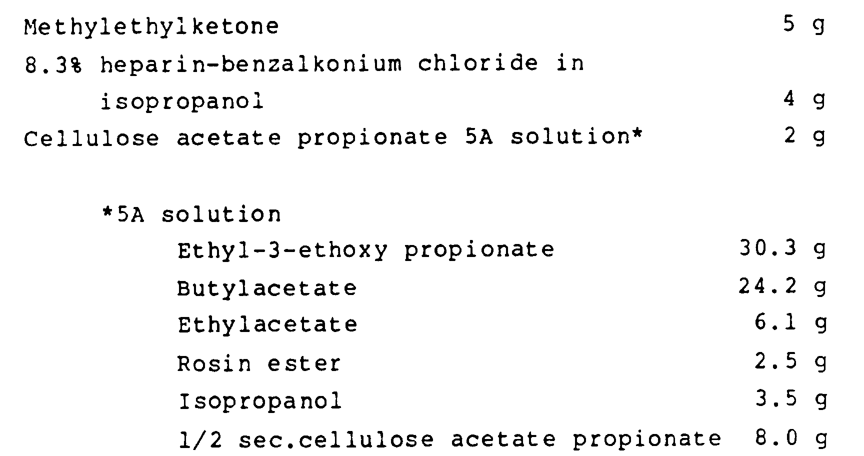

The coated sample was extracted in plasma at 37°C for four hours and tested for anti-microbial activity by pressing it into gelled Difco Plate Agar which was spiked with Staphylococcus epidermidis (ATCC 12228) and then incubated overnight at 32-35°C. A sample of polyurethane tubing that was coated with heparin-benzalkonium chloride without cellulose polymer was extracted in plasma at 37°C for four hours for comparison. The sample which contained cellulose acetate propionate (CAP) polymer showed a significant zone of inhibition while the sample made without CAP resin showed no zone of inhibition, demonstrating that the incorporation of cellulose ester polymer effectively increases resistance to removal of the coating when extracted in human plasma.

### Example 6

Example 5 was repeated, except that the solution contained 1.5 gm of 10.7% (wt. %) nitrocellulose solution in place of the 2.0 grams of 10.7% (wt. %) CAP solution. Samples of polyurethane tubing coated with this solution were extracted in plasma at 37°C for four hours or 18 hours. They were then tested for anti-microbial activity using the same zone of inhibition test as used in Example 5. The tests showed zones of inhibition after both extraction intervals. The sample extracted for four hours has a larger zone of inhibition than the sample that was extracted for 18 hours.

### Example 7

The following solution was coated on polyurethane 7 French tubing and dried at 80°C for 20 minutes. A control was made by coating a sample of the tubing with a 5% w/v solution of Tridodecylmethylammonium chloride (TDMAC).

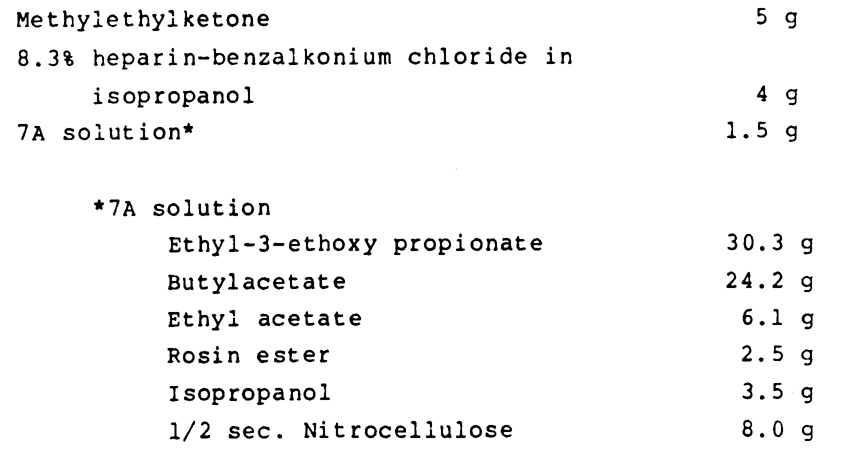

Both samples were then immersed for 30 minutes in a 5% aqueous solution of penicillin G and then air dried overnight. The coated samples were then extracted for 18 hours in human plasma at 37°C. They were removed from the plasma, rinsed in running deionized water and then tested for anti-microbial activity as in Example 5. The sample containing nitrocellulose showed a strong zone of inhibition while the sample without nitrocellulose showed no zone of inhibition.

### Example 8

Example 7 was repeated, except that TDMAC was added to the coating solutions as follows:

| | |
|---|---|
| Example 8 | .025 gm TDMAC added |
| Example 8A | .075 gm TDMAC added |

Both samples showed a strong zone of inhibition after the 18 hours plasma extraction and appeared to be substantially comparable to Example 7.

### Example 9

Polyurethane 7 French tubing was coated with the following solution.

| | |
|---|---|
| Heparin tridodecylmethylammonium chloride | 0.2 g |
| Isopropanol | 2.6 g |
| Methylethylketone | 2.5 g |
| 7A Solution | 0.7 g |

This coated sample was tested for clotting and did not clot. It was very resistant to removal in hot running water.

### Example 10

Polyurethane 7 French tubing was coated with a solution containing the following ingredients and dried at ambient temperature for 60 minutes:

| | |
|---|---|
| Methylethylketone | 5.3 g |
| Heparin-benzalkonium chloride | 0.31 g |
| Isopropanol | 3.4 g |
| Acrylic resin | 0.2 g |
| Rosin ester | 0.2 g |
| Tridodecylmethylammonium chloride | 0.4 g |
| Xylene | 0.14 g |
| Butanol | 0.05 g |

Samples were then extracted in plasma at 37°C for 4, 24 and 120 hours and compared to uncoated polyurethane tubing for anti-clotting activity. The results were as follows:

| Sample | Clotting Time |
|---|---|
| Uncoated control | 9 minutes |
| Above sample, without extraction in plasma | Did Not Clot |
| Above sample, after 4 hours extraction in plasma | Did Not Clot |
| Above sample, after 24 hours extraction in plasma | Did Not Clot |
| Above sample, after 120 hours extraction in plasma | Did Not Clot |

The above coated sample was resistant to removal by hot running water.

### Example 11

Polyurethane 7 French tubing was coated with a solution containing the following ingredients and dried 15 minutes at 75°C:

| | |
|---|---|
| Methylethylketone | 5.6 g |
| Heparin-benzalkonium chloride | 0.33 g |
| Isopropanol | 3.5 g |
| Polyurethane resin | 0.24 g |
| Polyisocyanate resin | 0.19 g |
| Ethyl acetate | 0.19 g |

Samples were extracted in plasma at 37°C for 72 hours and then tested for anti-clotting properties. A sample of polyurethane tubing which was coated with heparin-benzalkonium chloride (1.8% w/v in isopropanol) was also extracted in plasma at 37°C for 72 hours for comparison. The following results were obtained:

| Sample | Clotting Time |
|---|---|
| Uncoated control | 13 minutes |
| Above sample, after 72 hours extraction in plasma | Did Not Clot |
| Sample coated with heparin-benzalkonium chloride in isopropanol, after 72 hours extraction in plasma | 7 minutes |

The above coating was also resistant to removal by hot running water.

### Example 12

Polyurethane 7 French tubing was coated with a solution containing the following ingredients and dried for 20 minutes at 70°C.

| | |
|---|---|
| Methylethylketone | 5.9 g |
| Heparin-benzalkonium chloride | 0.32 g |
| Isopropanol | 3.5 g |
| Polyurethane resin | 0.14 g |
| Polyisocyanate resin | 0.07 g |
| Ethylacetate | 0.07 g |

Samples were then extracted in human plasma at 37°C for 3, 24, and 48 hours and then tested for anti-clotting properties. The following results were obtained:

| Sample | Clotting Time |
|---|---|
| Uncoated control | 8 minutes |
| Above sample, after 3 hours extraction in plasma | Did Not Clot |
| Above sample, after 24 hours extraction in plasma | Did Not Clot |
| Above sample, after 48 hours extraction in plasma | 9 minutes |

### Example 13

Polyurethane 7 French tubing was coated with a solution containing the following ingredients and dried for 20 minutes at 70°C.

| | |
|---|---|
| Methylethylketone | 6.1 g |
| Heparin-benzalkonium chloride | 0.32 g |
| Isopropanol | 3.5 g |
| Polyurethane resin | 0.07 g |
| Polyisocyanate resin | 0.04 g |
| Ethylacetate | 0.04 g |

Coated tubing was then extracted in plasma for 3 and 24 hours and then tested for anti-clotting behavior. The following results were obtained:

| Sample | Clotting Time |
|---|---|
| Uncoated control | 8 minutes |
| Above sample, after 3 hours extraction in plasma | Did Not Clot |
| Above sample, after 24 hours extraction in plasma | 9 minutes |

### Example 14

Polyurethane 7 French tubing was coated with a solution containing the following ingredients and dried for 20 hours at 55°C.

| | |
|---|---|
| Heparin tridodecylmethylammonium chloride | 0.32 g |
| Dimethylacetamide | 6.2 g |
| Toluene | 2.0 g |
| Petroleum ether | 1.5 g |

The coated tubing was extracted in human plasma at 37°C for 1, 2, 3 and 6 days and then tested for anti-clotting properties.

| Sample | Clotting Time |
|---|---|
| Uncoated sample | 10 minutes |
| Above sample, after 1 day extraction in plasma | Did Not Clot |
| Above sample, after 2 days extraction in plasma | Did Not Clot |
| Above sample, after 3 days extraction in plasma | Did Not Clot |
| Above sample, after 6 days extraction in plasma | Did not Clot |

The preceding examples, together with controls, show clearly that heparin-quaternary ammonium compounds and other pharmaceutical agents that are not polymeric can be made more resistant to removal or deactivation in various body fluids such as whole blood or plasma (including human) by mixing with appropriate water-insoluble polymers. Coatings made from normal heparin-quaternary ammonium compounds by themselves using solvents that do not cause mixing with the substrate, such as heparin-benzalkonium chloride, or heparin tridodecylmethylammonium chloride show little anti-thrombogenicity after soaking in human plasma for only a few hours. The heparin-TDMAC compound continues to show anti-thrombogenicity somewhat longer than the benzalkonium chloride compound, but both exhibit almost no anti-thrombogenicity after soaking in human plasma for a few hours. The incorporation of water-insoluble polymers according to the Present invention, and as shown in the examples, greatly extends the time for which coating samples can be soaked in human plasma and still show substantially levels of anti-thrombogenicity. For instance, some samples were found to show anti-thrombogenicity even after soaking in human plasma for 28 days.

On the other hand, when quaternary ammonium polymers are reacted with heparin, the coating remains on the surface even after long periods of soaking in body fluids such as human plasma, but the anti-thrombogenicity is not as strong either before soaking or after soaking for up to 28 days in human plasma, as in the samples made according to this invention. It is further noted that by water-insoluble polymers we are implying that they are water-insoluble after a film is cast and dried, and include water-insoluble polymers that may be hydrophilic, but nevertheless cause the heparin-quaternary ammonium compounds to remain anti-thrombogenic after prolonged soaking in body fluids.

It has been found that it is possible to react an antibiotic or other pharmaceutical agent such as penicillin, ticarcillin, cefotoxin, cephalosporins, oxacillin, and carbonicillin that contains a positive inorganic ion such as sodium with a quaternary ammonium compound such as benzalkonium chloride or (TDMAC) to produce an ionic antibiotic that is soluble in organic solvents and is miscible with hydrophobic water insoluble polymers. In this case, the resulting polymer mixture would not contain an anti-thrombogenic agent such as heparin. It is also possible to react other antibiotics or pharmaceutical agents that contain a negative ion such as chloride with surfactants that contain a negative organic ion such as sodium laurylsulfate to again convert a water soluble antimicrobial agent or other pharmaceutical agent into one that is soluble in organic solvents and miscible with hydrophobic water insoluble polymers. In accordance with the invention, it is possible to incorporate pharmaceutical agents without reaction with ionic surfactants if the pharmaceutical agent has low water solubility and is soluble in organic solvents and miscible with the water insoluble cellulose ester polymers of the present invention. When these organic solvent soluble agents are mixed with polymers of this invention, they can be rendered much more resistant to removal in plasma from the surface of an article coated with them than if they are coated on the surface without the polymer.

By using antibiotics or other pharmaceutical agents that are soluble in organic solvents, or by making the antibiotic or other pharmaceutical agent soluble in organic solvents and miscible with the water insoluble cellulose ester polymers of this invention, it makes it possible to incorporate useful pharmaceuticals such as antibiotics onto medical devices at the time of manufacture. The pharmaceuticals are available at the surface of the device in efficacious concentrations, over a useful period such as several days to weeks. At the same time, while the pharmaceuticals are present in useful concentrations where they are wanted on the device surface, they are not present in high concentration systematically so that typical side effects normally associated with various pharmaceuticals are unlikely.

The polymer can be mixed with the pharmaceutical agent and then coated, or the polymer or agent can be coated first and then overcoated with the other agent. The single coating of a mixture of polymer and pharmaceutical agent would normally be preferred because it would involve only a single coating and because the ratio of pharmaceutical agent to polymer can be controlled more precisely. Such mixtures of pharmaceutical agents and polymers would not be anti-thrombogenic unless they also contained an anti-thrombogenic agent such as heparin. However, the coatings do show strongly the desired effect of the incorporated pharmaceutical agent such as anti-microbial activity. The presence of certain polymers also has the added benefit of enhancing the stability of the pharmaceutical agent to a sterilization process such as exposure to ethylene oxide.

The pharmaceutical agent is preferably present in a concentration of about 0.5% to 99.5% by weight with the balance preferably comprising the water-insoluble polymer. The concentration of the water-insoluble polymer is typically about 0.01% to 40% by weight and the concentration of antimicrobial-surfactant compound is about 0.01% to 40% by weight of the coating solution.

The following example demonstrates how the system works.

In Examples 15-18, ¹⁴C-penicillin G sodium salt was reacted with tridodecylmethylammonium chloride (TDMAC) using procedures similar to those previously described in the Background Of The Invention, see A. Amplatz, "A Simple Non-Thrombogenic Coating", Invest. Radiology, July, August, 1971, Vol. 6.

A typical method of preparation for the pharmaceutical agent-TDMAC compounds of the present invention is as follows:

Seventeen grams TDMAC is dissolved in 60 ml isopropanol, and diluted with 40 ml distilled water. Next, dissolve 10 grams of the sodium salt of the pharmaceutical agent (SPA) in 100 ml distilled water. Mix equal volumes of both liquids and shake vigorously for ten or so seconds to ensure complete mixing and reaction.

Next, vacuum filter over filter paper, collect the compound off the paper, and place in a centrifuge jar with 1 volume of water, shake for 30 minutes, and vacuum filter again on filter paper. The wash is repeated twice more. The SPA-TDMAC is dried in an oven at 60°C.

Using this basic procedure, it is obvious to one skilled in the art that organic salts can be made from many or most ionic pharmaceutical agents by mixing them together with an appropriate ionic surfactant, and washing out the water-soluble salt residue with water. These compounds are soluble in organic solvents and typically have very low solubility constants so that when mixed with the polymers of this invention, constant and efficacious concentrations of the pharmaceutical agent (5) will be available on the coated surface in vivo over an extended period.

The resultant ¹⁴C-penicillin-TDMAC prepared by the above method is soluble in various organic solvents and has extremely low water solubility but is still ionic. The ¹⁴C-pencillin-TDMAC was then mixed with selected polymers and coated on both silicone and polyurethane tubing. The coatings were then extracted in plasma for one day or 5 days and compared to non-extracted samples by scintillation counting to determine how much penicillin remained on the surface in the coating after extraction. Some samples were exposed to an ethylene oxide sterilization cycle and tested by zone of inhibition, to show whether the polymer improved the resistance of the antibiotic to degradation when exposed to ethylene oxide.

### Example 15

The ¹⁴C-penicillin-TDMAC was mixed with cellulose nitrate dissolved in a solvent mixture containing ethanol, isopropanol, ethylacetate and toluene. The solution has the following composition:

| | |
|---|---|
| Nitrocellulose | 1.8 g |
| Isopropanol | .8 g |
| Toluene | 24.3 g |
| Ethyl acetate | 5.1 g |
| Camphor | .5 g |
| Dibutylphthalate | .7 g |
| ¹⁴C-penicillin-TDMAC | 2.0 g |

The solution was coated on both silicone and polyurethane tubing and dried. Some coated samples were then extracted in plasma for 24 hours or five days. After plasma extraction, the samples were measured by scintillation counting and were compared to unextracted samples to show how much ¹⁴C-penicillin-TDMAC remained. The following results were obtained.

| | Not Extracted | After 24 hrs. Extraction | After Five Days Extraction |
|---|---|---|---|
| Silicone tubing | 48 µg/cm² | 30 µg/cm² | 6 µg/cm² |
| Polyurethane tubing | 36 µg/cm² | 43 µg/cm² | 38 µg/cm² |

When the ¹⁴C-penicillin-TDMAC was coated without polymer, it was removed from the tubing surface in a few hours. These results clearly show how incorporation of this polymer into the coating dramatically extends the elution time from the surface when extract in plasma.

### Example 16

Example 15 was repeated using Silastic silicone resin in 1,1,1,-Trichloroethane in place of the nitrocellulose solution. The solution has the following composition:

| | |
|---|---|
| Silastic Polymer | 1.3 g |
| 1,1,1-Trichloroethane | 28.7 g |
| Toluene | 8.0 g |
| ¹⁴C-penicillin-TDMAC | 2.0 g |

This sample was tested for resistance to extraction in plasma, and for resistance to degradation by ethylene oxide sterilization.

Results:

| | Not Extracted | After 24 hrs. Extraction | After Five Days Extraction |
|---|---|---|---|
| Silicone tubing | 68 µg/cm² | 33 µg/cm² | 27 µg/cm² |
| Polyurethane tubing | 18 µg/cm² | 8 µg/cm² | 6 µg/cm² |

These results show that incorporation of Silasticβ resin into the coating extends the elution time of the antibiotic in plasma to several days compared to a few hours without the resins.

After exposure to a typical ethylene oxide sterilization (ETO) cycle, the samples were tested by classic zone of inhibition testing. This was done by placing a sample (sterilized or non-sterilized) onto a layer of agar containing bacteria and then incubated. The results are reported as the size in mm of the clear zone surrounding the coated article which results from the antimicrobial activity of active ¹⁴C-penicillin-TDMAC.

| | Before ETO Exposure | After ETO Exposure |
|---|---|---|
| With Silasticβ | 11 | 10 |
| Without polymer | 26 | 0 |

This result clearly demonstrates how incorporation of Silasticβ polymer into the ¹⁴C-penicillin-TDMAC coating greatly increases the resistance of the antibiotic to degradation caused by exposure to ethylene oxide.

### Example 17

Example 15 was repeated using polyvinylbutyral (PVB) polymer in toluene in place of the nitrocellulose solution. The solution has the following composition:

| | |
|---|---|
| Polyvinylbutryral | 1.5 g |
| Toluene | 31.5 g |
| ¹⁴C-penicillin-TDMAC | 2.0 g |

This was coated on silicone and polyurethane tubings, dried, and then tested for resistance to extraction in plasma, and resistance to degradation during an ethylene oxide sterilization by zone of inhibition. The following results were obtained.

| | Not Extracted | After 24 hrs. Extraction | After Five Days Extraction |
|---|---|---|---|
| Silicone tubing | 18 µg/cm² | 18 µg/cm² | 16 µg/cm² |
| Polyurethane tubing | 12 µg/cm² | 13 µg/cm² | 10 µg/cm² |

| | Before ETO Sterilization | After ETO Sterilization |
|---|---|---|
| With PVB Polymer | 25 mm | 15 mm |
| Without Polymer | 26 mm | 0 |

Clearly, PVB polymer provides significant stabilization to ¹⁴C-penicillin-TDMAC against degradation caused by exposure to ethylene oxide.

### Example 18

Example 1 was repeated using cellulose acetate butyrate polymer (CAB) in place of the nitrocellulose solution. The solution has the following composition:

| | |
|---|---|
| Cellulose Acetate Butyrate | 2.0 g |
| Ethyl acetate | 8.0 g |
| Toluene | 17.0 g |
| ¹⁴C-penicillin-TDMAC | 2.0 g |

This solution was coated on silicone and polyurethane tubings, dried, and tested for resistance to extraction from the surface in plasma. It was also tested for resistance to degradation from ethylene oxide exposure. The following results were obtained.

| | Not Extracted | After 24 hrs. Extraction | After Five Days Extraction |
|---|---|---|---|
| Silicone tubing | 33 µg/cm² | 12 µg/cm² | 17 µg/cm² |
| Polyurethane tubing | 20 µg/cm² | 12 µg/cm² | 7 µg/cm² |

CAB polymer clearly increases the resistance of ¹⁴C-penicillin-TDMAC to extraction in plasma.

| | Before ETO Sterilization | After ETO Sterilization |
|---|---|---|
| With CAB Polymer | 31 mm | 31 mm |
| Without Polymer | 26 mm | 0 |

This result shows how CAB polymer provides substantial stabilization to ¹⁴C-penicillin-TDMAC against ETO induced degradation.

The foregoing Examples show how incorporation of these and/or other water insoluble polymers clearly improves resistance of pharmaceutical agents or pharmaceutical salts of organic ions to extraction in plasma and against the degradation effects from exposure to sterilization with ethylene oxide. At the same time, however, the incorporation of polymers still leaves effective concentrations of antibiotic or other pharmaceutical agent available at the coated surface as demonstrated by the zone of inhibition test results in Examples 16, 17 and 18.

It is expected that different polymers could be used together in a single solution/coating or in contiguous layers to further enhance performance and achieve specific results. We have also tested other polymers mixed with the pharmaceutical agents or organic ion salts of pharmaceuticals and found similarly useful improvement in resistance to extraction by plasma. These include polyisocyanates, acrylicpolymers, vinylacetate, and others.

Examples 19, 20, 21 and 22 demonstrate further how various drugs can be incorporated into coatings of the invention and are suitable for use on medical devices.

### Example 19

The following solution was made and coated on 1/4" (6.4 mm) lD, 1/16" 2.2 mm) wall thickness polyurethane rings and dried at 80°C.

| | |
|---|---|
| Cellulose acetate butyrate | 0.35 g |
| Norfloxacin | 0.17 g |
| Benzylalcohol | 1.58 g |
| Toluene | 1.57 g |
| Dimethylacetamide | 5.25 g |
| Butyl Acetate | 1.58 g |

Coated rings were then extracted in artificial urine at 37°C and then tested for zone of inhibition vs. E. coli. The coating was still effective up to 35 days in the artificial urine. Coated samples sterilized by EtO were also effective against E. coli.

### Example 20

2 ml. sodium methotrexate (25 mg/ml) was placed in a test tube and 4 ml. Ethanol was added. The methotrexate precipitated out of solution. Tridodecylmethylammonium chloride was added and the test tube was swirled to mix the agents. The methotrexate quickly went into solution as the tridodecylmethylammonium salt. This mixture was shaken with an equal volume of toluene to separate the water and sodium chloride from the methotrexate tridodecylmethylammonium salt. The toluene layer separated on top and had the characteristic yellow color of methotrexate salts. The aqueous layer was clear and had no color. The toluene layer was diluted with an equal volume of 2% cellulose acetate butyrate in Butyrolactone. This was coated on a polyurethane catheter surface and produced a clear layer.

### Example 21

Three drops of 7.4% solution of gentamicin chloride in 62.5% water, 37.5% dimethylacetamide was diluted with 15 drops of glacial acetic acid, and 1.5 ml ethanol. Next, three drops of nitrocellulose in butyrolactone was added. This solution was clear, and produced a clear layer when coated and dried on glass.

### Example 22

The following solution was coated on glass and dried for 2 minutes at 80°C.

| | |
|---|---|
| Merbarone | 0.1 gm |
| Dimethysulfoxide | 1.98 gm |
| Cellulose acetate butyrate | 0.12 gm |
| Ethanol | 2.0 gm |

This solution was clear, and the dried layer on glass was also clear.

## Claims

1. A pharmaceutical composition comprising a pharmaceutical agent which has not been reacted with any ionic surfactant and a water-insoluble cellulose ester polymer which is miscible with the pharmaceutical agent and is at least one selected from non-ether cellulose esters, cellulose nitrate, cellulose acetate, cellulose acetate butyrate and cellulose acetate propionate, the polymer and the pharmaceutical agent having been co-dissolved in a mutual solvent or solvent mixture, whereby the composition maintains efficacious activity levels of the pharmaceutical agent for an extended period of time.

2. A composition according to claim 1, further comprising a hydrophilic polymer.

3. A composition according to claim 2, in which the hydrophilic polymer is polyvinylpyrrolidone.

4. A composition according to any preceding claim, in which the pharmaceutical agent is at least one selected from antithrombogenic agents, antibiotic agents and anti-cancer agents.

5. A composition according to claim 4, in which the pharmaceutical agent is an antibiotic agent selected from quinolones, aminoglycosides, cephalosporins, sulfonoamides and tetracyclines and/or an anti-cancer agent selected from methotrexate and merbarone.

6. A composition according to any preceding claim, in which the water-insoluble polymer further comprises at least one selected from polyurethane resins including polyester and polyether types, acrylic polymers, condensation polymers, isocyanates and polyacetals.

7. A composition according to any preceding claim, the concentrations of the pharmaceutical agent and the water-insoluble cellulose ester polymer in the solvent or solvent mixture being from 0.1 to 20% by weight and from 0.01 to 20% by weight, respectively.

8. A medical device having at least a portion of its surface coated by a coating composition comprising a pharmaceutical agent which has not been reacted with any ionic surfactant and a water-insoluble cellulose ester polymer which is miscible with the pharmaceutical agent, whereby the coating composition maintains efficacious activity levels of the pharmaceutical agent for an extended period of time.

9. A device according to claim 8, in which the coating composition further comprises a hydrophilic polymer.

10. A device according to claim 9, in which the hydrophilic polymer is polyvinylpyrrolidone.

11. A device according to any of claims 8 to 10, in which the pharmaceutical agent is at least one selected from antithrombogenic agents, antibiotic agents and anti-cancer agents.

12. A device according to claim 11, in which the pharmaceutical agent is an antibiotic agent selected from guinolones, aminoglycosides, cephalosporins, sulfonoamides and tetracyclines and/or an anti-cancer agent selected from methotrexate and merbarone.

13. A device according to any of claims 8 to 12, in which the waer-insoluble polymer further comprises at least one selected from polyurethane resins including polyester and polyether types, acrylic polymers, condensation polymers isocyanates and polyacetals.

14. A device according to any of claims 8 to 13, in which the water-insoluble cellulose ester polymer is at least one selected from cellulose nitrate, cellulose acetate, cellulose acetate butyrate and cellulose acetate propionate.

15. A device according to any of claims 8 to 14, in which the pharmaceutical agent is present in a concentration of about 0.5% to 99.5% by weight with the balance comprising the water-insoluble polymer.

16. A device according to any of claims 8 to 15, in which the water-insoluble polymer and the pharmaceutical agent are present in separate layers in which inter-layer molecular mingling exists at least across the interface, if not deeper.

17. A method of forming a coating on the surface of a medical device to be brought into contact with human or animal body fluids, the method comprising
(a) providing a coating solution which comprises a pharmaceutical agent which has not been reacted with any ionic surfactant and a water-insoluble cellulose ester polymer which is miscible with the pharmaceutical agent, the polymer and the pharmaceutical agent having been co-dissolved in a mutual solvent or solvent mixture,
(b) applying the coating solution to the surface of the device, and
(c) allowing the solution to dry.

18. A method of forming a coating on the surface of a medical device to be brought into contact with human or animal body fluids, the method comprising
(a) providing
(i) a first coating solution comprising a pharmaceutical agent which has not been reacted with any ionic surfactant and a solvent or solvent mixture therefor and
(ii) a second coating solution comprising a water-insoluble cellulose ester polymer which is miscible with the pharmaceutical agent and a solvent or solvent mixture therefor
(b) applying the first and second coating solutions separately to the surface of the device, and
(c) allowing the solutions to dry.

19. A method of forming a coating on the surface of a water-insoluble cellulose ester polymer, the method comprising
(a) providing a coating solution which comprises a pharmaceutical agent which has not been reacted with any ionic surfactant and a solvent or solvent mixture therefor
(b) applying the coating solution to a surface of the water-insoluble cellulose ester polymer, and
(c) allowing the solution to dry.

20. A method according to claim 17 or 18, in which the concentration of the water-insoluble cellulose ester polymer in its respective coating solution is 0.01% to 20% by weight of the solution.

21. A method according to claim 17, 18 or 20, in which the coating solution containing the water-insoluble polymer further comprises at least one selected from polyurethane resins including polyester and polyether types, acrylic polymers, condensation polymers, isocyanates and polyacetals.

22. A method according to claim 17, 18, 20 or 21, in which the pharmaceutical agent is present in the final coating in a concentration of about 0.5% to 99.5% by weight with the balance comprising the water-insoluble polymer.

23. A method according to claim 17, 18, 20, 21 or 22, in which the water-insoluble polymer and the pharmaceutical agent are present in the final coating in separate layers in which inter-layer molecular mingling exists at least across the interface, if not deeper.

24. A method according to any of claims 17 to 23, in which the concentration of the pharmaceutical agent in its respective coating solution is 0.1% to 20% by weight of the solution.

25. A method according to any of claims 17 to 24, in which the pharmaceutical agent is at least one selected from antithrombogenic agents, antibiotic agents and anti-cancer agents.

26. A method according to claim 25, in which the pharmaceutical agent is an antibiotic agent selected from quinolones, aminoglycosides, cephalosporins, sulphonoamides and tetracyclines and/or an anti-cancer agent selected from methotrexate and merbarone.

27. A method according to any of claims 17 to 26, in which the water-insoluble cellulose ester polymer is at least one selected from cellulose nitrate, cellulose acetate, cellulose acetate butyrate and cellulose acetate propionate.

28. A method according to any of claims 17 to 27, in which the or at least one coating solution further comprises a hydrophilic polymer.

29. A method according to claim 28, in which the hydrophilic polymer is polyvinylpyrrolidone.

30. A medical device having at least a portion of its surface coated by a method according to any of claims 17 to 29.

31. A medical device according to any of claims 8 to 15 and claim 30 when dependent on any of claims 17, 18, 20, 21, 22 and 23, in which the coated portion is at least one of glass, metal and plastic.

32. A medical device according to any of claims 8 to 16, 30 and 31, the device being a catheter, a fluid drainage device, a suction or aspiration device or an artificial blood vessel, heart or kidney.

## Patentansprüche

1. Pharmazeutische Zusammensetzung, umfassend ein pharmazeutisches Mittel, das nicht mit einem ionischen oberflächenaktiven Mittel umgesetzt worden ist, und ein wasserunlösliches Celluloseester-Polymeres, das mit dem pharmazeutischen Mittel mischbar ist, wobei es sich um mindestens einen unter Nichtether-Celluloseestern, Cellulosenitrat, Celluloseacetat, Celluloseacetobutyrat und Celluloseacetopropionat ausgewählten Bestandteil handelt, wobei das Polymere und das pharmazeutische Mittel gemeinsam in einem für beide Bestandteile geeigneten Lösungsmittel oder Lösungsmittelgemisch gelöst sind, wobei die Zusammensetzung für eine längere Zeitspanne wirksame Aktivitätsspiegel des pharmazeutischen Mittels aufrechterhält.

2. Zusammensetzung nach Anspruch 1, ferner umfassend ein hydrophiles Polymeres.

3. Zusammensetzung nach Anspruch 2, wobei es sich beim hydrophilen Polymeren um Polyvinylpyrrolidon handelt.

4. Zusammensetzung nach einem der vorstehenden Ansprüche, wobei es sich beim pharmazeutischen Mittel um mindestens einen unter antithrombogenen Mitteln, antibiotischen Mitteln und Antikrebsmitteln ausgewählten Bestandteil handelt.

5. Zusammensetzung nach Anspruch 4, wobei es sich beim pharmazeutischen Mittel um ein unter Chinolonen, Aminoglycosiden, Cephalosporinen, Sulfonamiden und Tetracyclinen ausgewähltes antibiotisches Mittel und/oder um ein unter Methotrexat und Merbaron ausgewähltes Antikrebsmittel handelt.

6. Zusammensetzung nach einem der vorstehenden Ansprüche, wobei das wasserunlösliche Polymere zusätzlich mindestens einen unter Polyurethanharzen unter Einschluß von Harzen vom Polyester- und Polyethertyp, Acrylpolymeren, Kondensationspolymeren, Isocyanaten und Polyacetalen ausgewählten Bestandteil umfaßt.

7. Zusammensetzung nach einem der vorstehenden Ansprüche, wobei die Konzentrationen des pharmazeutischen Mittels und des wasserunlöslichen Celluloseester-Polymeren im Lösungsmittel oder Lösungsmittelgemisch 0,1 bis 20 Gew.-% bzw. 0,01 bis 20 Gew.-% betragen.

8. Medizinische Vorrichtung, bei der mindestens ein Teil ihrer Oberfläche mit einer Überzugsmasse beschichtet ist, die ein pharmazeutisches Mittel, das nicht mit einem ionischen oberflächenaktiven Mittel umgesetzt worden ist, und ein wasserunlösliches Celluloseester-Polymeres umfaßt, das mit dem pharmazeutischen Mittel mischbar ist, wobei die Überzugsmasse für eine längere Zeitspanne wirksame Aktivitätsspiegel des pharmazeutischen Mittels aufrechterhält.

9. Vorrichtung nach Anspruch 8, wobei die Überzugsmasse ferner ein hydrophiles Polymeres umfaßt.

10. Vorrichtung nach Anspruch 9, wobei es sich beim hydrophilen Polymeren um Polyvinylpyrrolidon handelt.

11. Vorrichtung nach einem der Ansprüche 8 bis 10, wobei es sich beim pharmazeutischen Mittel um mindestens einen unter antithrombogenen Mitteln, antibiotischen Mitteln und Antikrebsmitteln ausgewählten Bestandteil handelt.

12. Vorrichtung nach Anspruch 11, wobei es sich beim pharmazeutischen Mittel um ein unter Chinolonen, Aminoglycosiden, Cephalosporinen, Sulfonamiden und Tetracyclinen ausgewähltes antibiotisches Mittel und/oder um ein unter Methotrexat und Merbaron ausgewähltes Antikrebsmittel handelt.

13. Vorrichtung nach einem der Ansprüche 8 bis 12, wobei das wasserunlösliche Polymere ferner mindestens einen unter Polyurethanharzen unter Einschluß von Harzen vom Polyester- und Polethertyp, Acrylpolymeren, Kondensationspolymeren, Isocyanaten und Polyacetalen ausgewählten Bestandteil umfaßt.

14. Vorrichtung nach einem der Ansprüche 8 bis 13, wobei es sich beim wasserunlöslichen Celluloseester-Polymeren um mindestens einen unter Cellulosenitrat, Celluloseacetat, Celluloseacetobutyrat und Celluloseacetopropionat ausgewählten Bestandteil handelt.

15. Vorrichtung nach einem der Ansprüche 8 bis 14, wobei das pharmazeutische Mittel in einer Konzentration von etwa 0,5 bis 99,5 Gew.-% vorhanden ist und der Rest das wasserunlösliche Polymere umfaßt.

16. Vorrichtung nach einem der Ansprüche 8 bis 15, wobei das wasserunlösliche Polymere und das pharmazeutische Mittel in getrennten Schichten vorliegen, bei denen eine Zwischenschicht-Molekülvermengung zumindest im Bereich der Grenzfläche - wenn nicht tiefer - vorliegt.

17. Verfahren zur Bildung eines Überzugs auf der Grenzfläche einer medizinischen Vorrichtung, die in Kontakt mit menschlichen oder tierischen Körperflüssigkeiten zu bringen ist, wobei das Verfahren folgendes umfaßt:
(a) Bereitstellen einer Beschichtungslösung, die ein pharmazeutisches Mittel, das nicht mit einem ionischen oberflächenaktiven Mittel umgesetzt worden ist, und ein wasserunlösliches Celluloseester-Polymeres umfaßt, das mit dem pharmazeutischen Mittel mischbar ist, wobei das Polymere und das pharmazeutische Mittel in einem für beide Bestandteile geeigneten Lösungsmittel oder Lösungsmittelgemisch gemeinsam in Lösung gebracht worden sind,
(b) Auftragen der Beschichtungslösung auf die Oberfläche der Vorrichtung und
(c) Trocknenlassen der Lösung.

18. Verfahren zur Bildung eines Überzugs auf der Oberfläche einer medizinischen Vorrichtung, die in Kontakt mit menschlichen oder tierischen Körperflüssigkeiten zu bringen ist, wobei das Verfahren folgendes umfaßt
(a) Bereitstellen
(i) einer ersten Beschichtungslösung, die ein pharmazeutisches Mittel, das nicht mit einem ionischen oberflächenaktiven Mittel umgesetzt worden ist, und ein Lösungsmittel oder Lösungsmittelgemisch dafür umfaßt, und
(ii) einer zweiten Beschichtungslösung, die ein wasserunlösliches Celluloseester-Polymeres, das mit dem pharmazeutischen Mittel mischbar ist, und ein Lösungsmittel oder Lösungsmittelgemisch hierfür umfaßt,
(b) getrenntes Auftragen der ersten und zweiten Beschichtungslösung auf die Oberfläche der Vorrichtung und
(c) Trocknenlassen der Lösungen.

19. Verfahren zur Bildung eines Überzugs auf der Oberfläche eines wasserunlöslichen Celluloseester-Polymeren, wobei das Verfahren folgendes umfaßt
(a) Bereitstellen einer Beschichtungslösung, die ein pharmazeutisches Mittel, das nicht mit ionischen oberflächenaktiven Mitteln umgesetzt worden ist, und ein Lösungsmittel oder Lösungsmittelgemisch hierfür umfaßt,
(b) Auftragen der Beschichtungslösung auf eine Oberfläche des wasserunlöslichen Celluloseester-Polymeren und
(c) Trocknenlassen der Lösung.

20. Verfahren nach Anspruch 17 oder 18, wobei die Konzentration des wasserunlöslichen Celluloseester-Polymeren in der jeweiligen Beschichtungslösung 0,01 bis 20 Gew.-% der Lösung beträgt.

21. Verfahren nach Anspruch 17, 18 oder 20, wobei die das wasserunlösliche Polymere enthaltende Beschichtungslösung ferner mindestens einen unter Polyurethanharzen unter Einschluß von Harzen vom Polyester- und Polyethertyp, Acrylpolymeren, Kondensationspolymeren, Isocyanaten und Polyacetalen ausgewählten Bestandteil umfaßt.

22. Verfahren nach Anspruch 17, 18, 20 oder 21, wobei das pharmazeutische Mittel im fertigen Überzug in einer Konzentration von etwa 0,5 bis 99,5 Gew.-% vorhanden ist und der Rest das wasserunlösliche Polymere umfaßt.

23. Verfahren nach Anspruch 17, 18, 20, 21 oder 22, wobei das wasserunlösliche Polymere und das pharmazeutische Mittel im fertigen Überzug in getrennten Schichten vorliegen, wobei eine Zwischenschicht-Molekülvermengung zumindest im Bereich der Grenzfläche - wenn nicht tiefer - vorliegt.

24. Verfahren nach einem der Ansprüche 17 bis 23, wobei die Konzentration des pharmazeutischen Mittels in der jeweiligen Beschichtungslösung 0,1 bis 20 Gew.-% der Lösung beträgt.

25. Verfahren nach einem der Ansprüche 17 bis 24, wobei es sich beim pharmazeutischen Mittel um mindestens einen unter antithrombogenen Mitteln, antibiotischen Mitteln und Antikrebsmitteln ausgewählten Bestandteil handelt.

26. Verfahren nach Anspruch 25, wobei es sich beim pharmazeutischen Mittel um ein unter Chinolonen, Aminoglycosiden, Cephalosporinen, Sulfonamiden und Tetracyclinen ausgewähltes antibiotisches Mittel und/oder um ein unter Methotrexat und Merbaron ausgewähltes Antikrebsmittel handelt.

27. Verfahren nach einem der Ansprüche 17 bis 26, wobei es sich beim wasserunlöslichen Celluloseester-Polymeren um mindestens einen unter Cellulosenitrat, Celluloseacetat, Celluloseacetobutyrat und Celluloseacetopropionat ausgewählten Bestandteil handelt.

28. Verfahren nach einem der Ansprüche 17 bis 27, wobei die Beschichtungslösung oder die mindestens eine Beschichtungslösung ferner ein hydrophiles Polymeres umfaßt.

29. Verfahren nach Anspruch 28, wobei es sich beim hydrophilen Polymeren um Polyvinylpyrrolidon handelt.

30. Medizinische Vorrichtung, bei der mindestens ein Teil ihrer Oberfläche durch ein Verfahren nach einem der Ansprüche 17 bis 29 beschichtet worden ist.

31. Medizinische Vorrichtung nach einem der Ansprüche 8 bis 15 und 30 unter Rückbeziehung auf einen der Ansprüche 17, 18, 20, 21, 22 und 23, wobei es sich beim beschichteten Bereich um mindestens einen der Bestandteile Glas, Metall und Kunststoff handelt.

32. Verfahren nach einem der Ansprüche 8 bis 16, 30 und 31, wobei es sich bei der Vorrichtung um einen Katheter, um eine Flüssigkeitsdrainagevorrichtung, eine Saug- oder Absaugvorrichtung oder ein künstliches Blutgefäß, ein künstliches Herz oder eine künstliche Niere handelt.

## Revendications

1. Composition pharmaceutique comprenant un agent pharmaceutique qui n'a pas été amené à réagir avec un tensio-actif ionique quelconque et un polymère d'ester cellulosique insoluble dans l'eau qui est miscible avec l'agent pharmaceutique et qui est au moins un choisi parmi les esters cellulosiques non éther, le nitrate de cellulose, l'acétate de cellulose, l'acétate et butyrate de cellulose et l'acétate et propionate de cellulose, le polymère et l'agent pharmaceutique ayant été co-dissous dans un solvant mutuel ou un mélange de solvants, ainsi la composition conserve des degrés d'activité efficaces de l'agent pharmaceutique pendant une durée prolongée.

2. Composition selon la revendication 1, comprenant de plus un polymère hydrophile.

3. Composition selon la revendication 2, dans laquelle le polymère hydrophile est la polyvinyl-pyrrolidone.

4. Composition selon l'une quelconque des revendications 1 à 3, dans laquelle l'agent pharmaceutique est au moins un choisi parmi les agents antithrombogènes, les agents antibiotiques et les agents anticancéreux.

5. Composition selon la revendication 4, dans laquelle l'agent pharmaceutique est un agent antibiotique choisi parmi les quinolones, les aminoglycosides, les céphalosporines, les sulfonoamides et les tétracyclines et/ou un agent anti-cancéreux choisi parmi le méthotrexate et la merbarone.

6. Composition selon l'une quelconque des revendications 1 à 5, dans laquelle le polymère insoluble dans l'eau comprend de plus au moins un choisi parmi les résines polyuréthanne incluant les types polyester et polyéther, les polymères acryliques, les polymères de condensation, les isocyanates et les polyacétals.

7. Composition selon l'une quelconque des revendications 1 à 6, les concentrations de l'agent pharmaceutique et du polymère d'ester cellulosique insoluble dans l'eau dans le solvant ou le mélange de solvants étant respectivement de 0,1 à 20% en poids et de 0,01 à 20% en poids.

8. Dispositif médical ayant au moins une partie de sa surface revêtue par une composition de revêtement comprenant un agent pharmaceutique qui n'a pas été amené à réagir avec un tensio-actif ionique quelconque et un polymère d'ester cellulosique insoluble dans l'eau qui est miscible avec l'agent pharmaceutique, ainsi la composition de revêtement maintient des degrés d'activité efficaces de l'agent pharmaceutique pendant une durée prolongée.

9. Dispositif selon la revendication 8, dans lequel la composition de revêtement comprend de plus un polymère hydrophile.

10. Dispositif selon la revendication 9, dans lequel le polymère hydrophile est la polyvinylpyrrolidone.

11. Dispositif selon l'une quelconque des revendications 8 à 10, dans lequel l'agent pharmaceutique est au moins un choisi parmi les agents antithrombogènes, les agents antibiotiques et les agents anticancéreux.

12. Dispositif selon la revendication 11, dans lequel l'agent pharmaceutique est un agent antibiotique choisi parmi les quinolones, les aminoglycosides, les céphalosporines, les sulfonoamides et les tétracyclines et/ou un agent anti-cancéreux choisi parmi le méthotrexate et la merbarone.

13. Dispositif selon l'une quelconque des revendications 8 à 12, dans lequel le polymère insoluble dans l'eau comprend de plus au moins un choisi parmi les résines polyuréthanne incluant les types polyester et polyéther, les polyméres acryliques, les polymères de condensation, les isocyanates et les polyacétals.

14. Dispositif selon l'une quelconque des revendications 8 à 13, dans lequel le polymère d'ester cellulosique insoluble dans l'eau est au moins un choisi parmi le nitrate de cellulose, l'acétate de cellulose, l'acétate et butyrate de cellulose et l'acétate et propionate de cellulose.

15. Dispositif selon l'une quelconque des revendications 8 à 14, dans lequel l'agent pharmaceutique est présent dans une concentration d'environ 0,5% à 99,5% en poids, le complément comprenant le polymère insoluble dans l'eau.

16. Dispositif selon l'une quelconque des revendications 8 à 15, dans lequel le polymère insoluble dans l'eau et l'agent pharmaceutique sont présents en couches séparés dans lesquelles l'entremêlement moléculaire inter-couches existe au moins à travers l'interface, sinon plus profondément.

17. Procédé de formation d'un revêtement sur la surface d'un dispositif médical à amener en contact avec des fluides corporels humains ou animaux, le procédé comprenant les étapes consistant:
(a) à fournir une solution de revêtement qui comprend un agent pharmaceutique qui n'a pas été amené à réagir avec un tensio-actif ionique quelconque et un polymère d'ester cellulosique insoluble dans l'eau qui est miscible avec l'agent pharmaceutique, le polymère et l'agent pharmaceutique ayant été co-dissous dans un solvant mutuel ou un mélange de solvants;
(b) à appliquer la solution de revêtement sur la surface du dispositif; et
(c) à laisser sécher la solution.

18. Procédé de formation d'un revêtement sur la surface d'un dispositif médical à amener en contact avec des fluides corporels humains ou animaux, le procédé comprenant les étapes consistant:
(a) à fournir
(i) une première solution de revêtement comprenant un agent pharmaceutique qui n'a pas été amené à réagir avec un tensio-actif ionique quelconque et un solvant ou mélange de solvants pour celui-ci;
(ii) une deuxième solution de revêtement comprenant un polymère d'ester cellulosique insoluble dans l'eau qui est miscible avec l'agent pharmaceutique et un solvant ou mélange de solvants pour celui-ci;
(b) à appliquer la première et la deuxième solutions de revêtement séparément à la surface du dispositif; et
(c) à laisser sécher les solutions.

19. Procédé de formation d'un revêtement sur la surface d'un polymère d'ester cellulosique insoluble dans l'eau, le procédé comprenant les étapes consistant:
(a) à fournir une solution de revêtement qui comprend un agent pharmaceutique qui n'a pas été amené à réagir avec un tensio-actif ionique quelconque et un solvant ou mélange de solvants pour celui-ci;
(b) à appliquer la solution de revêtement à une surface du polymère d'ester cellulosique insoluble dans l'eau; et
(c) à laisser sécher la solution.

20. Procédé selon la revendication 17 ou 18, dans lequel la concentration en le polymère d'ester cellulosique insoluble dans l'eau dans sa solution de revêtement respective est de 0,01% à 20% en poids de la solution.

21. Procédé selon la revendication 17, 18 ou 20, dans lequel la solution de revêtement contenant le polymère insoluble dans l'eau comprend de plus au moins un choisi parmi les résines polyuréthanne incluant les types polyester et polyéther, les polymères acryliques, les polymères de condensation, les isocyanates et les polyacétals.

22. Procédé selon la revendication 17, 18, 20 ou 21, dans lequel l'agent pharmaceutique est présent dans le revêtement final à une concentration d'environ 0,5% à 99,5% en poids, le complément comprenant le polymère insoluble dans l'eau.

23. Procédé selon la revendication 17, 18, 20, 21 ou 22, dans lequel le polymère insoluble dans l'eau et l'agent pharmaceutique sont présents dans le revêtement final en couches séparées dans lesquelles l'entremêlement moléculaire inter-couches existe au moins à travers l'interface, sinon plus profondément.

24. Procédé selon l'une quelconque des revendications 17 à 23, dans lequel la concentration en l'agent pharmaceutique dans sa solution de revêtement respective est de 0,1% à 20% en poids de la solution.

25. Procédé selon l'une quelconque des revendications 17 à 24, dans lequel l'agent pharmaceutique est au moins un choisi parmi les agents antithrombogènes, les agents antibiotiques et les agents anti-cancéreux.

26. Procédé selon la revendication 25, dans lequel l'agent pharmaceutique est un agent antibiotique choisi parmi les quinolones, les aminoglycosides, les céphalosporines, les sulfonoamides et les tétracyclines et/ou l'agent anti-cancéreux choisi parmi le méthotrexate et la merbarone.

27. Procédé selon l'une quelconque des revendications 17 à 26, dans lequel le polymère d'ester cellulosique insoluble dans l'eau est au moins un choisi parmi le nitrate de cellulose, l'acétate de cellulose, l'acétate et butyrate de cellulose et l'acétate et propionate de cellulose.

28. Procédé selon l'une quelconque des revendications 17 à 27, dans lequel la ou au moins une solution de revêtement comprend de plus un polymère hydrophile.

29. Procédé selon la revendication 28, dans lequel le polymère hydrophile est la polyvinylpyrrolidone.

30. Dispositif médical ayant au moins une portion de sa surface revêtue par un procédé selon l'une quelconque des revendications 17 à 29.

31. Dispositif médical selon l'une quelconque des revendications 8 à 15 et la revendication 30 quand il dépend de l'une quelconque des revendications 17, 18, 20, 21, 22 et 23, dans lequel la portion revêtue est au moins une de verre, de métal et de matière plastique.

32. Dispositif médical selon l'une quelconque des revendications 8 à 16, 30 et 31, le dispositif étant un cathéter, un dispositif de drainage de fluide, un dispositif de succion ou d'aspiration ou un vaisseau sanguin, un coeur ou un rein artificiels.
